(19) Europäisches Patentamt
European Patent Office
Office européen des brevets

(11) Veröffentlichungsnummer: **0 161 602**
**B1**

(12) # EUROPÄISCHE PATENTSCHRIFT

(45) Veröffentlichungstag der Patentschrift: **04.10.89**

(21) Anmeldenummer: **85105482.5**

(22) Anmeldetag: **06.05.85**

(51) Int. Cl.⁴: **C 07 D 263/58,** C 07 D 277/68, C 07 D 413/12, C 07 D 417/12, A 01 N 43/74

(54) **6-Chlorbenzazolyloxyacetamide.**

(30) Priorität: **16.05.84 DE 3418168**

(43) Veröffentlichungstag der Anmeldung: **21.11.85 Patentblatt 85/47**

(45) Bekanntmachung des Hinweises auf die Patenterteilung: **04.10.89 Patentblatt 89/40**

(84) Benannte Vertragsstaaten: **AT BE CH DE FR GB IT LI NL SE**

(56) Entgegenhaltungen:
**EP-A-0 005 501**
**DE-A-3 038 599**
**DE-A-3 038 652**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk (DE)**

(72) Erfinder: **Förster, Heinz, Dr., Am Eckbusch 47, D-5600 Wuppertal 1 (DE)**
Erfinder: **Mues, Volker, Dr., Gellertweg 13, D-5600 Wuppertal 1 (DE)**
Erfinder: **Schmidt, Robert R., Dr., Im Waldwinkel 110, D-5060 Bergisch- Gladbach 2 (DE)**
Erfinder: **Santel, Hans- Joachim, Dr., Gerstenkamp 19, D-5000 Köln 80 (DE)**
Erfinder: **Hänssler, Gerd, Dr., Heymannstrasse 40, D-5090 Leverkusen 1 (DE)**
Erfinder: **Eue, Ludwig, Dr., Paul- Klee- Strasse 36, D-5090 Leverkusen 1 (DE)**

**EP 0 161 602 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Die Erfindung betrifft neue 6-Chlorbenzazolyloxyacetamide, ein Verfahren zu ihrer Herstellung und ihre Verwendung als Herbizide und Fungizide.

Es ist bereits bekannt, daß bestimmte Benzazolyloxyacetamide, wie beispielsweise das 2-(Benzthiazol-2-yl)-N-methyl-oxyacetanilid, herbizide, insbesondere auch selektiv-herbizide Eigenschaften besitzen (vgl. z. B. DE-A-2 903 966 bzw. EP-A-5 501). Darüber hinaus sind auch bestimmte 5-Chlorbenzoxazolyloxyacetamide als Herbizide bekannt geworden (vgl. DE-A-3 038 652).

Die herbizide Wirkung dieser vorbekannten Verbindungen gegenüber Schadpflanzen ebenso wie ihre Verträglichkeit gegenüber wichtigen Kulturpflanzen ist jedoch insbesondere bei niedrigen Aufwandmengen und Konzentrationen nicht immer in allen Anwendungsbereichen völlig zufriedenstellend. Über eine fungizide Wirkung der vorbekannten Benzazolyloxyacetamide ist bisher nichts bekannt.

Es wurden neue 6-Chlorbenzazolyloxyacetamide der allgemeinen Formel (I) gefunden,

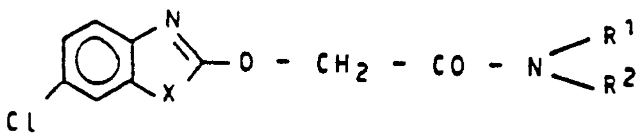

(I)

in welcher

X für Sauerstoff oder Schwefel steht und

$R^1$ und $R^2$ unabhängig voneinander für geradkettiges oder verzweigtes Alkyl mit 1 bis 6 Kohlenstoffatomen, für geradkettiges oder verzweigtes Alkenyl und Alkinyl mit jeweils 2 bis 6 Kohlenstoffatomen, für gegebenenfalls ein- bis dreifach, gleich oder verschieden durch Methyl oder Ethyl substituiertes Cycloalkyl und Cycloalkenyl mit 5 bis 7 Kohlenstoffatomen, für jeweils geradkettiges oder verzweigtes Alkoxy, Alkoxyalkylenoxy oder Alkoxyalkyl mit jeweils 1 bis 6 Kohlenstoffatomen in den einzelnen Alkylteilen, für Halogenalkyl mit 1 bis 6 Kohlenstoffatomen und 1 bis 5 Halogenatomen, insbesondere Fluor, Brom und Chlor, für Benzyl sowie für gegebenenfalls ein- bis dreifach, gleich oder verschieden durch Methyl, Ethyl, Methoxy, Methylthio, Trifluormethyl, Trifluormethoxy, Trifluormethylthio, Fluor, Chlor oder Nitro substituiertes Phenyl stehen;

oder

$R^1$ und $R^2$ zusammen mit dem Stickstoffatom, an das sie gebunden sind, für einen gegebenenfalls ein- bis dreifach, gleich oder verschieden durch Methyl, Ethyl oder Phenyl substituierten Heterocyclus der Formel

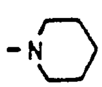 ; 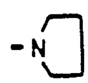 ; 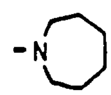 ; 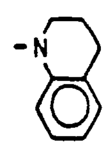

stehen

oder für den Heterocyclus der Formel

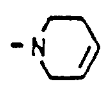

stehen.

Weiterhin wurde gefunden, daß man die neuen 6-Chlorbenzazolyloxyacetamide der allgemeinen Formel (I) erhält, wenn man 6-Chlorbenzazole der Formel (II),

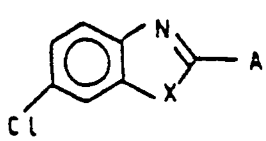

(II)

in welcher

X die oben angegebene Bedeutung hat und

A für eine elektronenanziehende Abgangsgruppierung steht,

mit Glykolsäureamiden der Formel (III)

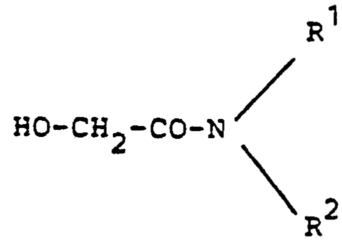

(III)

in welcher

$R^1$ und $R^2$ die oben angegebene Bedeutung haben,

gegebenenfalls in Gegenwart eines Verdünnungsmittels und gegebenenfalls in Gegenwart eines Säurebindemittels sowie gegebenenfalls in Gegenwart eines Katalysators umsetzt.

Schließlich wurde gefunden, daß die neuen 6-Chlorbenzazolyloxyacetamide der Formel (I) herbizide, insbesondere auch selektiv-herbizide Eigenschaften und darüberhinaus auch fungizide Eigenschaften besitzen.

Überraschenderweise zeigen die neuen 6-Chlorbenzazolyloxyacetamide der Formel (I) eine erheblich verbesserte herbizide Wirksamkeit gegen verbreitete, schwer bekämpfbare Unkräuter bei vergleichbar hoher Verträglichkeit gegenüber wichtigen Kulturpflanzen und gleichzeitig auch eine bessere fungizide Wirksamkeit im Vergleich zu den aus dem Stand der Technik vorbekannten Benzazolyloxyacetamiden, wie beispielsweise das N-Methyl-2-benzthiazolyloxyacetanilid, welche chemisch und wirkungsmäßig naheliegende Verbindungen sind.

Im einzelnen seien außer den bei den Herstellungsbeispielen genannten Verbindungen die folgenden Verbindungen der allgemeinen Formel (I) genannt:

Tabelle 1

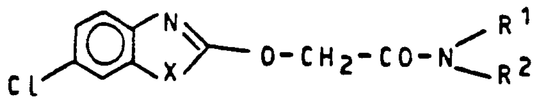

(I)

| X | $R^1$ | $R^2$ | bzw. 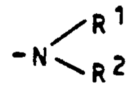 |
|---|---|---|---|
| O | $CH_3$ | $CH_3O-CH_2-$ | |
| O | $CH_3$ | 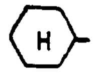 | |
| O | $CH_3$ | 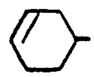 | |
| O | $CH_3$ | $F_3C-CH_2-$ | |
| O | $CH_3$ | 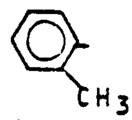 | |
| O | $CH_3$ | 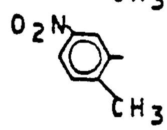 | |
| O | $CH_3$ | 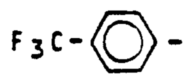 | |
| O | $C_2H_5$ | $CH_2=CH-CH_2-$ | |
| O | | | 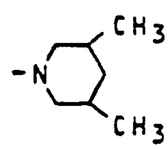 |
| O | | | 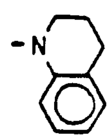 |
| O | | | 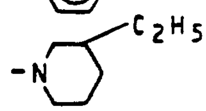 |

Tabelle 1 (Fortsetzung)

| X | $R^1$ | $R^2$ bzw. 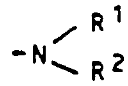 |
|---|---|---|
| O | $CH_3$ | $HC\equiv C-CH_2-$ |
| O | $CH_3O$ | 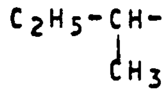 |
| O | $CH_3$ | 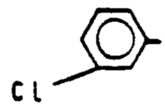 |
| O | $CH_3$ | 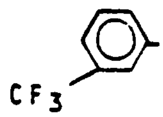 |
| S | $CH_3$ | 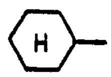 |
| S | $CH_3$ | 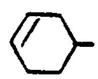 |
| S | $CH_3$ | $F_3C-CH_2-$ |
| S | $CH_3$ | 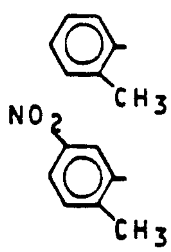 |
| S | $CH_3$ | |

Tabelle 1 (Fortsetzung)

| X | $R^1$ | $R^2$ | bzw. 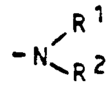 |
|---|---|---|---|
| S | $CH_3$- | 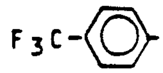 | |
| S | $CH_3O$- | 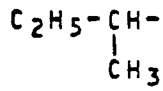 | |
| S | $(CH_3)_2CHO$- | $(CH_3)_2CH$- | |
| S | $C_2H_5$ | $CH_2=CH-CH_2$- | |
| S | | | 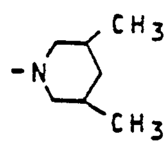 |
| S | | | 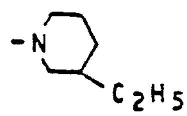 |
| O | | | 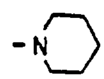 |
| S | | | 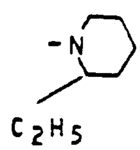 |
| O | | | 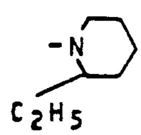 |
| O | $CH_3$- | $CH_2=CH-CH_2$- | |
| S | $CH_3$- | $CH_2=CH-CH_2$- | |

Tabelle 1 (Fortsetzung)

| X | $R^1$ | $R^2$ bzw. 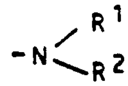 |
|---|---|---|
| O | $CH_3O-CH_2-$ | $C_2H_5-CH-$<br>\|<br>$CH_3$ |
| S | $CH_3O-CH_2-$ | $C_2H_5-CH-$<br>\|<br>$CH_3$ |
| O | $CH_3(CH_2)_3-$ | $CH_3(CH_2)_3-$ |
| O | $C_2H_5-$ | $(CH_3)_2CH-$ |
| S | $C_2H_5-$ | $(CH_3)_2CH-$ |
| S | $(CH_3)_2CH-$ | $C_2H_5OCH_2CH_2-O-$ |
| O | $CH_3-$ | 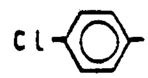 |
| S | $CH_3-$ | 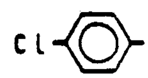 |
| S | $CH_3-$ | 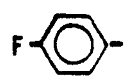 |
| S | $CH_3-$ | 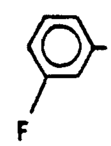 |
| O | $CH_3-$ | 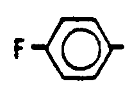 |
| O | $CH_3-$ | 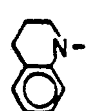 |
| S | $CH_3-$ | 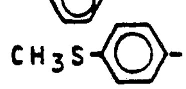 |
| O | $CH_3-$ | 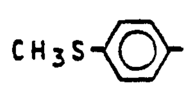 |
| S | $CH_3-$ | 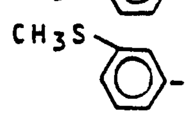 |

Tabelle 1 (Fortsetzung)

| X | $R^1$ | $R^2$ bzw. | 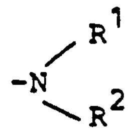 |
|---|---|---|---|
| S | $CH_3$- | 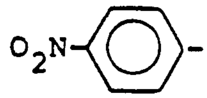 | |
| O | $CH_3$- | 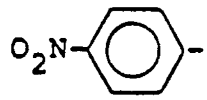 | |
| S | $CH_3$- | n-$H_9C_4$- | |
| O | | | 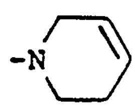 |
| O | $C_2H_5$- | $CH_2$=CH- | |

Verwendet man beispielsweise 2,6-Dichlorbenzoxazol und Glykolsäure-N-methylanilid als Ausgangsstoffe, so läßt sich der Reaktionsablauf des erfindungsgemäßen Verfahrens durch das folgende Formelschema darstellen:

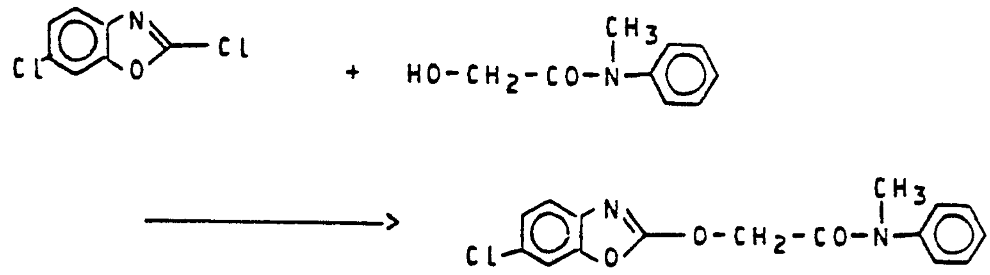

Die zur Durchführung des erfindungsgemäßen Verfahrens als Ausgangsstoffe benötigten 6-Chlorbenzazole sind durch die Formel (II) allgemein definiert. In dieser Formel (II) steht X für Sauerstoff oder Schwefel, A steht vorzugsweise für Halogen, insbesondere Chlor oder Brom, oder für Alkylsulfonyl bzw. Aralkylsulfonyl, insbesondere für Methylsulfonyl oder Ethylsulfonyl.

Die 6-Chlorbenzazole sind bekannt (vgl. z. B. EP-A-43 573 bzw. DE-A-3 025 910) oder lassen sich nach prinzipiell bekannten Methoden in einfacher analoger Weise herstellen.

Die zur Durchführung des erfindungsgemäßen Verfahrens weiterhin als Ausgangsstoffe benötigten Glykolsäureamide sind durch die Formel (III) allgemein definiert. Die Glykolsäureamide der Formel (III) sind ebenfalls bekannt (vgl. z. B. DE-A-2 904 490, EP-A-5 501, EP-A-29 171, DE-A-3 038 598, DE-A-3 244 956).

Als Verdünnungsmittel für das erfindungsgemäße Verfahren kommen organische oder anorganische Lösungsmittel in Frage. Bevorzugt sind Kohlenwasserstoffe, wie Toluol oder Cyclohexan, Halogenkohlenwasserstoffe, wie Methylenchlorid, Chloroform, Dichlorethan oder Chlorbenzol, Ketone, wie Aceton oder Methylisobutylketon, Ether, wie Diethylether, Diisopropylether oder Methyl-t-butylether, Alkohole, wie Methanol, Ethanol oder Isopropanol, Amide, wie Dimethylformamid oder Dimethylacetamid, Sulfoxide, wie Dimethylsulfoxid, Wasser oder wäßrige Salzlösungen.

Als Salze verwendet man hierbei vorzugsweise Chloride oder Sulfate von Alkali- oder Erdalkalimetallen, wie beispielsweise Natriumchlorid, Kaliumchlorid oder Calciumchlorid. Besonders bevorzugt ist Natriumchlorid.

Das erfindungsgemäße Verfahren wird vorteilhaft unter Verwendung von Säurebindemitteln durchgeführt. Als solche werden vorzugsweise stark basische Alkali- und Erdalkalimetallverbindungen, beispielsweise Oxide, wie z. B. Natrium-, Kalium-, Magnesium- und Calciumoxid, Hydroxide, wie z. B. Natrium-, Kalium-, Magnesium- und Calciumhydroxid und/oder Carbonate, wie z. B. Natrium-, Kalium-, Magnesium- und Calciumcarbonat verwendet.

Der Zusatz von 0,01 bis 10 Gew.-% (bezogen auf eingesetztes Glykolsäureamid der Formel (III)) eines Phasentransferkatalysators mag sich in einigen Fällen als vorteilhaft erweisen. Als Beispiele für solche Katalysatoren seien genannt:

Tetrabutylammoniumchlorid, Tetrabutylammoniumbromid, Tributyl-methylphosphoniumbromid, Trimethyl-$C_{13}/C_{15}$-alkyl -ammoniumchlorid, Dibenzyl-dimethyl-ammoniummethylsulfat, Dimethyl-$C_{12}/C_{14}$-alkyl-benzylammoniumchlorid, Tetrabutylammoniumhydroxid, 18-Krone-6, Triethylbenzylammoniumchlorid, Trimethylbenzylammoniumchlorid, Tetraethylammoniumbromid.

Die Reaktionstemperaturen können bei dem erfindungsgemäßen Verfahren innerhalb eines größeren Bereiches variiert werden. Sie liegen im allgemeinen zwischen -50°C und +100°C, vorzugsweise zwischen -20°C und +100°C.

Das erfindungsgemäße Verfahren wird im allgemeinen bei Normaldruck durchgeführt, es kann aber auch bei erhöhtem oder vermindertem Druck, etwa zwischen 0,1 und 10 bar, durchgeführt werden.

Zur Durchführung des erfindungsgemäßen Verfahrens setzt man pro Mol 6-Chlorbenzazol der Formel (II) im allgemeinen 0,1 bis 10 Mol, vorzugsweise 0,8 bis 1,2 Mol an Glykolsäureamid der Formel (III) und 0,5 bis 10 Mol, vorzugsweise 0,5 bis 3 Mol an Base ein. Die Zugabereihenfolge der Reaktanten kann beliebig vertauscht werden, es können auch alle Komponenten gleichzeitig in das Reaktionsgefäß eindosiert werden. Die Reaktionsführung kann kontinuierlich oder diskontinuierlich gestaltet werden. Die Aufarbeitung erfolgt in üblicher Art und Weise.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z. B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca,

Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z. B. auf Industrie- und Gleisanlagen und auf Wegen und Plätzen mit und ohne Baumbewuchs. Ebenso können die Verbindungen zur Unkrautbekämpfung in Dauerkulturen, z. B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei zeigen die erfindungsgemäßen Wirkstoffe neben einer hervorragenden Wirkung gegenüber Schadpflanzen auch eine gute Verträglichkeit gegenüber wichtigen Kulturpflanzen und können daher als selektive Unkrautbekämpfungsmittel in monokotylen Kulturen, wie Getreide und Reis ebenso wie in dikotylen Kulturen, wie Sojabohnen, Baumwolle, Zuckerrüben und anderen eingesetzt werden.

Darüber hinaus weisen die erfindungsgemäßen Wirkstoffe in entsprechenden Aufwandmengen auch eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Reiskrankheiten wie beispielsweise gegen den Erreger der Reisfleckenkrankheit (Pyricularia oryzae) einsetzen. Neben einer protektiven Wirksamkeit zeigen die erfindungsgemäßen Wirkstoffe hierbei insbesondere auch systemische Eigenschaften.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyethylen-Fettsäure-Ester, Polyoxyethylen-Fettalkohol-Ether, z. B. Alkylarylpolyglykol-Ether, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additve können mineralische und vegetabile Öle sein.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Bei der Verwendung als Herbizide können die erfindungsgemäßen Wirkstoffe als solche oder in ihren

Formulierungen auch in Mischung mit bekannten Herbiziden zur Unkrautbekämpfung Verwendung finden, wobei Fertigformulierungen und Tankmischungen möglich sind.

Für die Mischung kommen bekannte Herbizide wie z. B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,2-dimethylethyl)-3-methyl-thio-1,2,4-triazin-5(4H)-on zur Unkrautbekämpfung in Sojabohen, in Frage. Auch Mischungen mit N,N-Dimethyl-N'-(4-chlor-3-methylphenyl)-harnstoff, N,N-Dimethyl-N'-(4-isopropylphenyl)-harnstoff, mit weiteren Triazindionen oder Pyridyl-phenoxy-propionsäuren sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Emulsionen, Suspensionen, Pulver, Pasten und Granulate, angewendet werden. Die Anwendung geschieht in üblicher Weise, z. B. durch Gießen, Tauchen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen bei der Anwendung als Herbizide zwischen 0,01 und 10 kg Wirkstoff pro Hektar Bodenfläche, vorzugsweise zwischen 0,05 und 5 kg pro ha.

Bei der Verwendung als Fungizide können die erfindungsgemäßen Wirkstoffe ebenfalls in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate, angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustrieren.

Bei der Behandlung von Pflanzenteilen können die Wirkstoffkonzentrationen in den Anwendungsformen in einem größeren Berich variiert werden. Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Die nachfolgenden Beispiele dienen zur weiteren Erläuterung der Erfindung.

**Herstellungsbeispiele:**

**Beispiel 1**

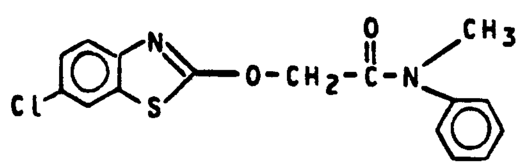

Zu einer Mischung von 3,3 g (0,02 Mol) Glykolsäure-N-methylanilid und 1,2 g (0,02 Mol) pulverisiertem Kaliumhydroxid in 50 ml Isopropanol gibt man bei -15°C eine Lösung von 4 g (0,02 Mol) 2,6-Dichlorbenzthiazol in 10 ml Acetonitril und rührt nach beendeter Zugabe weitere 15 Stunden bei -5°C. Zur Aufarbeitung gießt man die Reaktionsmischung in Wasser, saugt den ausgefallenen Feststoff ab, wäscht mit Wasser nach, trocknet und kristallisiert in einem Gemisch aus Essigsäure/Petrolether (1 : 1) um. Man erhält 4 g (75 % der Theorie) an 2-(6-Chlorbenzthiazol-2-yl)-N-methyl-oxyacetanilid vom Schmelzpunkt 108°C.

**Beispiel 2**

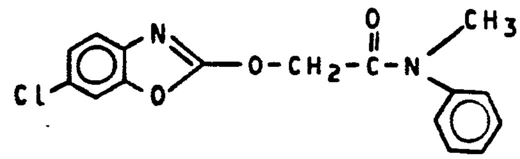

Zu einer Mischung von 8,2 g (0,05 Mol) Glykolsäure-Nmethylanilid und 3,1 g (0,05 Mol) pulverisiertem Kaliumhydroxid in 80 ml Isopropanol gibt man bei -10° C tropfenweise unter Rühren eine Lösung von 9,4 g (0,05 Mol) 2,6-Dichlorbenzoxazol in 20 ml Isopropanol und rührt nach beendeter Zugabe weitere 15 Stunden bei -5° C. Zur Aufarbeitung gießt man die Reaktionsmischung in Wasser, saugt den ausgefallenen Feststoff ab, wäscht mit Wasser nach und trocknet.

Man erhält 13 g (82 % der Theorie) an 2-(6-Chlorbenzoxazol-2-yl)-N-methyl -oxyacetanilid vom Schmelzpunkt 139° C.

In entsprechender Weise und gemäß den allgemeinen Herstellungsangaben erhält man die folgenden Verbindungen der allgemeinen Formel (I)

**Tabelle 2**

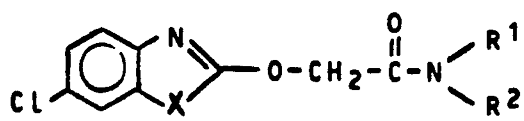

(I)

| Bsp.Nr. | $R^1$ | $R^2$ | bzw. $-NR^1R^2$ | X | Schmelzpunkt |
|---|---|---|---|---|---|
| 3 | $CH_3$ | $CH_3(CH_2)_3-$ | | O | 78-79°C |
| 4 | | | 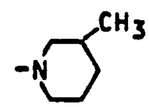 | O | 112°C |
| 5 | | | 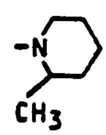 | O | 82°C |
| 6 | | | 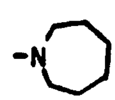 | O | 118°C |

Tabelle 2 (Fortsetzung)

| Bsp.Nr. | $R^1$ | $R^2$ | bzw. $-N\genfrac{}{}{0pt}{}{R^1}{R^2}$ | X | Schmelzpunkt |
|---|---|---|---|---|---|
| 23 | $CH_3$ | $CH{\equiv}C-CH_2-$ | | S | 115°C |
| 24 | | | 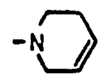 | S | 142°C |
| 25 | | | 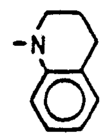 | S | 162°C |
| 26 | $CH_3$ | $CH_3(CH_2)_2-$ | | S | |
| 27 | $CH_3$ | $CH_3OCH_2-$ | | S | 77°C |
| 28 | $CH_3$ | 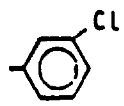 | | S | 123°C |
| 29 | $CH_3$ | 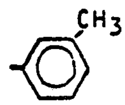 | | S | 126°C |
| 30 | $CH_3(CH_2)_3-$ | $CH_3(CH_2)_3-$ | | S | 67°C |
| 31 | $C_2H_5-$ | $CH_2{=}CH-$ | | S | 78°C |
| 32 | $CH_2{=}CH-CH_2-$ | $CH_2{=}CH-CH_2-$ | | O | 82°C |
| 33 | $(CH_3)_2CH-$ | $C_2H_5O-CH_2-CH_2-O-$ | | O | $n_D^{20}=1{,}5274$ |
| 34 | | | 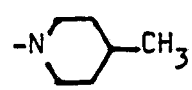 | O | 98°C |

**Anwendungsbeispiele**

In den nachfolgenden Anwendungsbeispielen wird die nachstehend aufgeführte Verbindung als Vergleichssubstanz herangezogen:

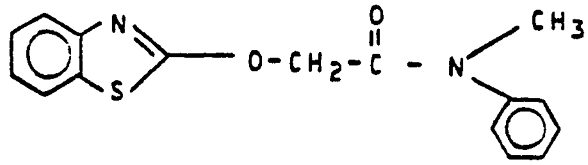

(A)

2-(Benzthiazol-2-yl)-N-methyl-oxyacetanilid (bekannt aus DE-A-2 903 966 bzw. EP-A-5 501)

In Beispiel A wird außerdem folgende Verbindung als Vergleichssubstanz herangezogen:

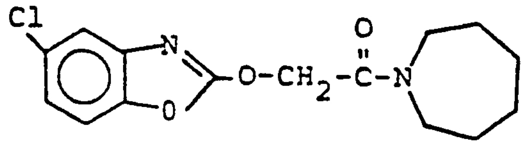

(B)

2-(5-Chlorbenzoxazol-2-yl)-N,N-hexamethylen-oxyacetamid (bekannt aus DE-A-3 038 652).

**Beispiel A**

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % =keine Wirkung (wie unbehandelte Kontrolle)
100 % =totale Vernichtung

Eine deutliche Überlegenheit in der herbiziden Wirksamkeit bei vergleichbarer Nutzpflanzenselektivität gegenüber dem Stand der Technik zeigen bei diesem Test z. B. die Verbindungen gemäß folgender Herstellungsbeispiele: 3 und 6.

**Beispiel B**

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % =keine Wirkung (wie unbehandelte Kontrolle)
100 % =totale Vernichtung

Eine deutliche Überlegenheit in der herbiziden Wirksamkeit bei vergleichbarer Nutzpflanzenselektivität gegenüber dem Stand der Technik zeigen bei diesem Test z. B. die Verbindungen gemäß folgender Herstellungsbeispiele: 6.

**Beispiel C**

Pyricularia-Test (Reis) / systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegeben Menge Emulgator auf die gewünschte Konzentration.

Zur Prüfung auf systemische Eigenschaften werden 40 ml der Wirkstoffzubereitung auf Einheitserde gegossen, in der jungen Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z. B. die Verbindungen gemäß folgender Herstellungsbeispiele: 3, 6 und 7.

**Patentansprüche**

1. 6-Chlorbenzazolyloxyacetamide der allgemeinen Formel (I)

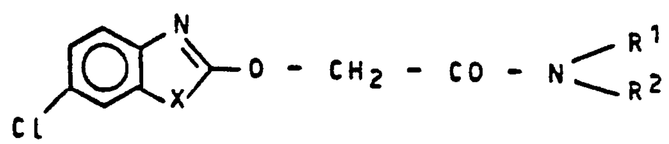

(I)

in welcher

X für Sauerstoff oder Schwefel steht und

$R^1$ und $R^2$ unabhängig voneinander für geradkettiges oder verzweigtes Alkyl mit 1 bis 6 Kohlenstoffatomen, für geradkettiges oder verzweigtes Alkenyl und Alkinyl mit jeweils 2 bis 6 Kohlenstoffatomen, für gegebenenfalls ein- bis dreifach, gleich oder verschieden durch Methyl oder Ethyl substituiertes Cycloalkyl und Cycloalkenyl mit 5 bis 7 Kohlenstoffatomen, für jeweils geradkettiges oder verzweigtes Alkoxy, Alkoxyalkylenoxy oder Alkoxyalkyl mit jeweils 1 bis 6 Kohlenstoffatomen in den einzelnen Alkylteilen, für Halogenalkyl mit 1 bis 6 Kohlenstoffatomen und 1 bis 5 Halogenatomen (insbesondere Fluor, Brom und Chlor), für Benzyl sowie für gegebenenfalls ein- bis dreifach, gleich oder verschieden durch Methyl, Ethyl, Methoxy, Methylthio, Trifluormethyl, Trifluormethoxy, Trifluormethylthio, Fluor, Chlor oder Nitro substituiertes Phenyl stehen;

oder

$R^1$ und $R^2$ zusammen mit dem Stickstoffatom, an das sie gebunden sind, für einen gegebenenfalls ein- bis dreifach, gleich oder verschieden durch Methyl, Ethyl oder Phenyl substituierten Heterocyclus der Formel

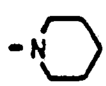 ; 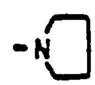 ; 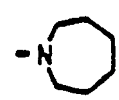 ; 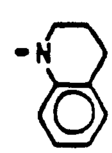

stehen oder für den Heterocyclus der Formel

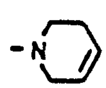

stehen

2. Verfahren zur Herstellung von 6-Chlorbenzazolyloxyacetamiden der allgemeinen Formel (I) gemäß Anspruch 1, dadurch gekennzeichnet, daß man 6-Chlorbenzazole der Formel (II)

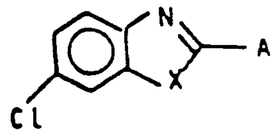

(II)

in welcher

X die in Anspruch 1 angegebene Bedeutung hat und
A für eine elektronenanziehende Abgangsgruppe steht,

mit Glykolsäureamiden der Formel (III)

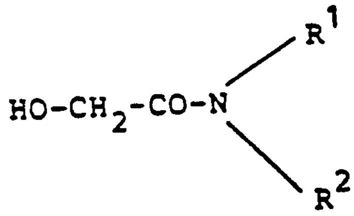

(III)

in welcher
$R^1$ und $R^2$ die in Anspruch 1 angegebene Bedeutung haben,
gegebenenfalls in Gegenwart eines Verdünnungsmittels und gegebenenfalls in Gegenwart eines Säurebindemittels sowie gegebenenfalls in Gegenwart eines Katalysators umsetzt.

3. Herbizide Mittel, gekennzeichnet durch einen Gehalt an mindestens einem 6-Chlorbenzazolyloxyacetamid der Formel (I) gemäß Anspruch 1.

4. Verwendung von 6-Chlorbenzazolyloxyacetamiden der allgemeinen Formel (I) gemäß Anspruch 1 zur Bekämpfung von unerwünschten Pflanzenwachstum.

5. Fungizide Mittel, gekennzeichnet durch einen Gehalt an mindestens einem 6-Chlorbenzazolyloxyacetamid der allgemeinen Formel (I) gemäß Anspruch 1.

6. Verwendung von 6-Chlorbenzazolyloxyacetamiden der allgemeinen Formel (I) gemäß Anspruch 1 zur Bekämpfung von unerwünschten Mikroorganismen.

7. Verfahren zur Herstellung von herbiziden und fungiziden Mitteln, dadurch gekennzeichnet, daß man 6-Chlorbenzazolyloxyacetamide der allgemeinen Formel (I) gemäß Anspruch 1 mit Streckmitteln und/oder oberflächenaktiven Mitteln vermischt.

8. a) 2-(6-Chlorbenzoxazol-2-yl)-N-methyl-N-n-butyl-oxyacetamid (3);
b) 2-(6-Chlorbenzoxazol-2-yl)-N,N-diethyl-oxyacetamid (7);
c) 2-(6-Chlorbenzoxazol-2-yl)-N,N-hexamethylen-oxyacetamid (6);
gemäß Anspruch 1.

## Claims

1. 6-Chlorobenzazolyloxyacetamides of the general formula (I)

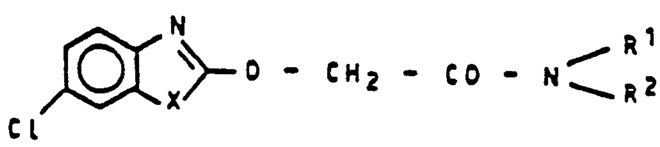

(I)

in which

X represents oxygen or sulphur and
$R^1$ and $R^2$ independently of one another represent straight-chain or branched alkyl with 1 to 6 carbon atoms, or represent straight-chain or branched alkenyl and alkinyl with in each case 2 to 6 carbon atoms, or represent cycloalkyl or cycloalkenyl which has 5 to 7 carbon atoms and is optionally mono-, di- or tri-substituted by identical or different radicals from the group comprising methyl and ethyl, or represent in each case straight-chain or branched alkoxy, alkoxyalkylenoxy or alkoxyalkyl with in each case 1 to 6 carbon atoms in the individual alkyl parts, or represent halogenoalkyl with 1 to 6 carbon atoms and 1 to 5 halogen atoms (in particular fluorine, bromine and chlorine), or represent benzyl, or represent phenyl which is optionally mono-, di- or trisubstituted by identical or different substituents from the series comprising methyl, ethyl, methoxy, methylthio, trifluoromethyl, trifluoromethoxy, trifluoromethylthio, fluorine, chlorine or nitro;

or

$R^1$ and $R^2$, together with the nitrogen atom to which they are bonded, represent a heterocyclic radical of the formula

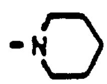 ; 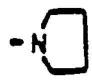 ; 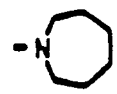 ; 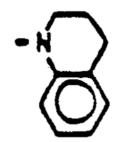

which is optionally mono-, di- or tri-substituted by identical or different substituents from the series comprising methyl, ethyl and phenyl or represent the heterocyclic radical of the formula

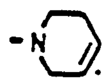.

2. Process for the preparation of 6-chlorobenzazolyloxyacetamides of the general formula (I) according to Claim 1, characterized in that 6-chlorobenzazoles of the formula (II)

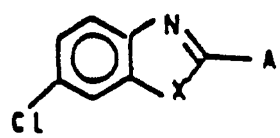

(II)

in which

X has the meaning given in Claim 1 and
A represents an electron-withdrawing leaving group,

are reacted with glycolic acid amides of the formula (III)

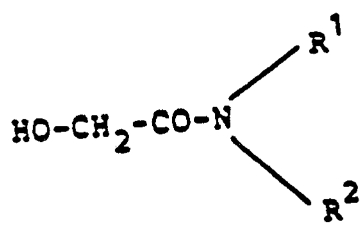

(III)

in which

$R^1$ and $R^2$ have the meaning given in Claim 1, if appropriate in the presence of a diluent and if appropriate in the presence of an acid-binding agent, and if appropriate in the presence of a catalyst.

3. Herbicidal agents, characterized in that they contain at least one 6-chlorobenzazolyloxyacetamide of the formula (I) according to Claim 1.

4. Use of 6-chlorobenzazolyloxyacetamides of the general formula (I) according to Claim 1 for combating undesirable plant growth.

5. Fungicidal agents, characterized in that they contain at least one 6-chlorobenzazolyloxyacetamide of the general formula (I) according to claim 1.

6. Use of 6-chlorobenzazolyloxyacetamides of the general formula (I) according to claim 1 for combating undesirable microorganisms.

7. Process for the preparation of herbicidal and fungicidal agents, characterized in that 6-chlorobenzazolyloxyacetamides of the general formula (I) according to claim 1 are mixed with extenders and/or surface-active agents.

8. a) 2-(6-Chlorobenzoxazol-2-yl)-N-methyl-N-n-butyloxyacetamide (3);
b) 2-(6-chlorobenzoxazol-2-yl)-N,N-diethyloxy-acetamide (7); and
c) 2-(6-chlorobenzoxazol-2-yl)-N,N-hexamethylene-oxyacetamide (6);

according to claim 1.

**Revendications**

1. 6-chlorobenzazolyloxy-acétamides de formule générale (I):

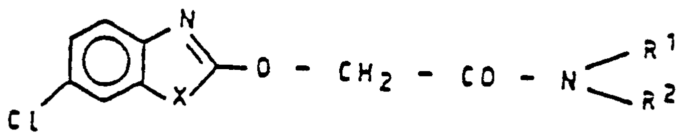

(I)

dans laquelle

X représente un atome d'oxygène ou un atome de soufre, et
$R^1$ et $R^2$ représentent chacun indépendamment l'un de l'autre, un groupe alkyle à chaîne droite ou ramifiée contenant 1 à 6 atomes de carbone, un groupe alcynyle et un groupe alcényle à chaîne droite ou ramifiée contenant chacun 2 à 6 atomes de carbone, un groupe cycloalkyle et un groupe cycloalcényle éventuellement substitué une à trois fois de manière identique ou différente par un groupe méthyle ou un groupe éthyle et contenant 5 à 7 atomes de carbone, un groupe alcoxy, un groupe alcoxyalkylénoxy ou un groupe alcoxyalkyle chaque fois à chaîne droite ou ramifiée et contenant chacun 1 à 6 atomes de carbone dans les fractions alkyle individuelles, un groupe halogénalkyle contenant 1 à 6 atomes de carbone et 1 à 5 atomes d'halogènes (en particulier, un atome de fluor, un atome de brome et un atome de chlore), un groupe benzyle, de même qu'un groupe phényle éventuellement substitué une à trois fois de manière identique ou différente par un groupe méthyle, un groupe éthyle, un groupe méthoxy, un groupe méthylthio, un groupe trifluorométhyle, un groupe trifluorométhoxy, un groupe trifluorométhylthio, un atome de fluor, un atome de chlore ou un groupe nitro;
ou
$R^1$ et $R^2$, ensemble avec l'atome d'azote auquel ils sont reliés, représentent un hétérocycle éventuellement substitué une à trois fois de manière identique ou différente par un groupe méthyle, un groupe éthyle ou un groupe phényle et répondant aux formules:

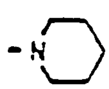 ; 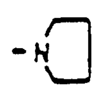 ; 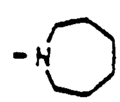 ; 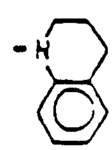

ou l'hétérocycle de formule:

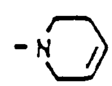

2. Procédé de préparation de 6-chloro-benzazolyloxy-acétamides de formule générale (I) selon la revendication 1, caractérisé en ce qu'on fait réagir des 6-chlorobenzazoles de formule (II):

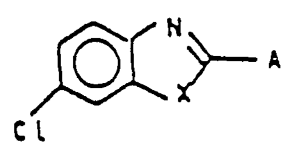

(II)

dans laquelle

X a la signification indiquée dans la revendication 1 et
A représente un groupe qui s'éloigne attirant les électrons,

avec des amides d'acide glycolique de formule (III):

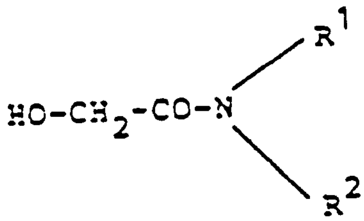

(III)

dans laquelle
$R^1$ et $R^2$ ont les significations indiquées dans la revendication 1,
éventuellement en présence d'un diluant éventuellement en présence d'un agent fixateur d'acide, ainsi qu'éventuellement en présence d'un catalyseur.

3. Agents herbicides, caractérisés en ce qu'ils contiennent au moins un 6-chlorobenzazolyloxy-acétamide de formule (I) selon la revendication 1.

4. Utilisation de 6-chlorobenzazolyloxy-acétamides de formule générale (I) selon la revendication 1, en vue de combattre la croissance de plantes adventices.

5. Agents fongicides, caractérisés en ce qu'ils contiennent au moins un 6-chlorobenzazolyloxy-acétamide de formule générale (I) selon la revendication 1.

6. Utilisation de 6-chlorobenzazolyloxyacét-amides de formule générale (I) selon la revendication 1 en vue de combattre des micro-organismes non désirés.

7. Procédé de préparation d'agents herbicides et fongicides, caractérisé en ce qu'on mélange des 6-chlorobenzazolyloxy-acétamides de formule générale (I) selon la revendication 1 avec des diluants et/ou des agents tensio-actifs.

8. a) le 2-(6-chlorobenzoxazol-2-yl)-N-méthyl-N-n-butyl-oxy-acétamide (3);
b) le 2-(6-chlorobenzoxazol-2-yl)-N,N-diéthyloxy-acétamide (7);
c) le 2-(6-chlorobenzoxazol-2-yl)-N,N-hexaméthylène-oxy-acétamide (6);

selon la revendication 1.